Europäisches Patentamt

European Patent Office

Office européen des brevets

(19)

(11) Publication number: **0 021 572**
**A1**

(12) # EUROPEAN PATENT APPLICATION

(21) Application number: **80301476.0**

(22) Date of filing: **06.05.80**

(51) Int. Cl.³: **C 12 Q 1/44**

(30) Priority: **04.06.79 US 45467**

(43) Date of publication of application:
**07.01.81 Bulletin 81/1**

(84) Designated Contracting States:
**AT BE CH DE FR GB IT LI LU NL SE**

(71) Applicant: **AMERICAN HOSPITAL SUPPLY CORPORATION**
**One American Plaza**
**Evanston, IL 60201(US)**

(72) Inventor: **Mazza, John Charles**
**8285 S.W. 205th Ter.**
**Miami, Florida 33189(US)**

(72) Inventor: **Dunka, Louis John**
**17741 S.W. 113th Ct.**
**Miami, Florida 33157(US)**

(74) Representative: **Seaborn, George Stephen et al,**
**c/o Edward Evans & Co. Chancery House 53-64 Chancery Lane**
**London WC2A 1SD(GB)**

(54) A substrate for use in a turbidimetric assay for lipase and a method for making this substrate.

(57) A novel substrate for use in turbidimetric assay for lipase is disclosed which contains a lyophilized emulsion of the lipase-hydrolysable triglyceride; a buffer; a surface active agent; and a bulking agent.

EP 0 021 572 A1

TITLE MODIFIED
see front page

- 1 -

## LIPASE SUBSTRATE

### Background of the Invention

Lipase is an enzyme secreted by the pancreas and is utilized in the digestion of triglycerides. Determination of lipase concentration in blood and other biological fluids is useful for the diagnosis of pancreatic dysfunction. The enzyme is an esterase which acts only on insoluble triglycerides, and the rate of lipase hydrolysis is dependent upon the surface area of the triglyceride available for contact with the enzyme. Thus, for effective enzyme action, both in vivo and in vitro, the triglyceride advantageously occurs as an aqueous emulsion. Lipase determinations are conventionally turbidimetric, titrametric, or colormetric. These techniques generally involve the preparation of an aqueous emulsion of a lipase-hydrolyzable triglyceride to which the lipase-containing sample is added. In a turbidimetric determination, the rate of decrease in turbidity is proportional to the lipase concentration in the sample. The substrate emulsion used in turbidimetric assays is usually buffered to maintain a pH conducive to optimum enzyme action. In titrametric assays, a basic solution is titrated into the substrate emulsion to neutralize free fatty acids as they are liberated by the enzyme. The rate of addition of base is thus proportional to the enzyme concentration. Colormetric assays generally involve color forming reactions with the enzyme-liberated glycerol or fatty acids.

Heretofore, turbidimetric techniques have involved preparation of a substrate emulsion by the analyst. Such emulsions are conventionally prepared by adding a predetermined amount of a suitable triglyderide, a surface active agent, and a buffer to water and agitating the mixture until a homogeneous emulsion is formed. The turbidity of the emulsion is dependent, to a large extent, upon the manner in which the emulsion is prepared. The procedure is time-consuming, and reproducible emulsions are difficult to obtain. Accordingly, there is a need for a ready-made, reproducible substrate emulsion with which an analyst can conduct rapid and accurate assays.

German Offenlegungsschrift 1 961 983 discloses a substrate for titrametric lipase determinations. The substrate is a lyophilized emulsion containing olive oil, a surface active agent, and a protective colloid. To use the substrate, the analyst reconstitutes the dry product with water, adds a sample aliquot thereto, and titrates the liberated fatty acids. Because the assay is titrametric, the emulsion contains a relatively large concentration of triglyceride, e.g. the ratio of olive oil to protective colloid is about 3:1.

Such high triglyceride concentrations, although preferred in titrametric assays, are undesirable in turbidimetric assays. Turbidimetric assays require relatively dilute emulsions of triglycerides to provide useful optical densities. Because turbidity is quite dependent upon the nature of the emulsion, reproducibility of the emulsion is more critical for turbidimetric substrates than for titrametric substrates. Ready-made, stable, turbidimetric substrates have not heretofore been prepared.

## Summary of the Invention

In accordance with the present invention, there is disclosed a substrate for use in a turbidimetric assay for lipase, which comprises a lyophilized emulsion of from about 0.5% to about 5 wt.% of a lipase-hydrolyzable triglyceride; a pH-controlling amount of a pH-7 to pH-10 buffer; from about 10 wt.% to about 40 wt.% of a surface active agent; and from about 20 wt.% to about 60 wt.% of a bulking agent. _

## Detailed Description of the Invention

The novel lyophilized lipase substrates of this invention are prepared from aqueous emulsions of a lipase-hydrolyzable triglyceride. Such triglycerides are generally fatty acid triesters of glycerol, in which the fatty acid substituents contain from about 3 to about 24 carbon atoms, preferably from about 16 to about 20 carbon atoms. Such fatty acids may be alphatic or olefinic. The triglycerides employed in such substrates may be purified materials such as triolein, tripalmitin, tristearin, trilinolein, stearo-diolein, palmito-oleosterin, and the like, or may be natural materials such as olive oil, peanut oil, sunflower seed oil, coconut oil, and the like. Olive oil is a preferred triglyceride for substrates of this invention, because of its suitability, availability, and comparatively low cost.

To prepare the aqueous emulsion from which the lyophilized substrate is made, a triglyceride-emulsifying surface active agent is employed. Any suitable surface active agent may be employed which does not deleteriously affect the other components of the substrate or interfere with the enzymatic reaction. Triglycerides are emulsified in vivo by the action of bile salts, such as physiological salts of desoxycholic acid, tauracholic acid, tauradesoxycholic acid, chenodesoxycholic acid, and the like, and these bile salts are particularly advantageous for the preparation of the substrates of this invention. Other synthetic or natural surface active agents, such as octylphenoxypolyethoxyethanol may be employed; however, certain synthetic detergents act as substrates for lipase, and such compounds should be avoided.

Generally, the lipase enzyme is most effective at a basic pH, e.g. a pH from about 7 to about 10. The preferred pH range for optimum enzyme action is from about 8 to about 9.5. Unlike titrametric assays in which pH is controlled within a narrow range by the constant addition of base, a turbidimetric assay generally utilizes a buffer to control the pH within an enzyme-hydrolyzing range. Accordingly, the substrate of this invention advantageously includes a pH-7 to pH-10 buffer. Any effective buffer which does not interfere with the enzyme reaction or deleteriously affect the substrate components may be used. A particularly preferred buffer is tris-(hydroxymethyl)-aminomethane. Other buffers which can be employed include zwitterion buffers such as N-tris(hydroxymethyl) methylglycine, glycinamide hydrochloride, N,N-bis(hydroxyethyl)glycine, and glycylglycine.

The above-identified components are vigorously blended in water to prepare a homogeneous stable emulsion. The concentrations of the various ingredients are controlled within general ranges and proportions to each other to provide such an emulsion. The concentrations of the ingredients in the aqueous emulsion are not critical, because the water will be removed during the lyophilization step, but the relative proportions of the components are important. Nevertheless, sufficient water is used to obtain desirable emulsion properties. Generally, sufficient water is employed to obtain a uniform emulsion. Any water that is used will be removed during lyophilization, therefore, excess water is usually avoided. The solids content of the aqueous emulsion will range from about the desired concentration of reconstituted lyophilized substrate to about five times that concentration. Preferred concentrations are from about 1 to about 3 times the reconstituted concentration of the substrate.

To prepare such emulsions, lipase-hydrolyzable triglyceride is added to water in an amount sufficient to produce an emulsion which, when diluted to the concentration to which the final lyophilized substrate will be reconstituted, has an absorbance of from about 0.3 to about 3.0 (Note: absorbance measurements were made at 405nm in a 1 cm cell versus a water blank on a Gilford Model 240 spectrophotometer (Gilford Instruments, Oberlin, Ohio). Absolute absorbance values may vary from instrument to instrument, but absorbance is useful for defining the general optical properties of the emulsion and is an accurate measurement of the reproducibility of emulsions). The optical absorbance of the emulsion is not solely dependent upon triglyceride concentration, but is also affected by such other factors as the degree of dispersion of the emulsion, and the particular ingredients used.

Triglyceride concentrations of from about 100 micromolar to about 425 micromolar are generally employed to obtain an absorbance within the desired range. Preferred concentrations of triglycerides are in the range of from about 200-300 micromolar.

The surface active agent is employed in a triglyceride-emulsifying amount, and this amount can vary considerably depending upon the surface active agent used. Generally, for sodium desoxycholate concentrations of surface active agent of from about 0.001 molar to 0.010 molar are employed, with preferred concentrations ranging from 0.004 molar to 0.008 molar.

The buffer is employed in a pH-controlling amount, and this amount can vary considerably depending upon the particular buffer used. For tris-(hydroxymethyl)-aminomethane buffer, a concentration of from about 0.015 molar to about 0.1 molar, preferably from about 0.020-0.075 molar is employed.

After the buffer is added to water, the pH is adjusted with acid within the range described above, and the other components of the emulsion are added, and the mixture is subjected to vigorous emulsification. Such emulsification may be accomplished by any effective means, such as high speed blending, hand homogenation, pressure homogenation, and ultrasonication. Frequently, two or more of these techniques are combined to produce a uniform emulsion. Preferred emulsification techniques are ultrasonication, in which the emulsion is subjected to high energy ultrasonic sound, and pressure homogenation, in which the emulsion is forced at high pressure through small orifices against a baffle plate. The emulsification is continued until the emulsion has an absorbance of from about 0.3 to about 3.0, preferably from about 0.5 to about 2.0. Emulsions may be prepared to a standard turbidity by blending appropriate emulsions of various absorbances.

A bulking agent is added to the emulsion to provide a matrix for lyophilization for the emulsion. The bulking agent may be any inert, water soluble compound. By inert is meant a compound which does not interfere with the enzyme reaction. Bulking agents which may be employed include hexitols such as mannitol and sorbitol, saccharides, such as sucrose, dextrose, etc., polysaccharides, such a gum acacia, guar gum, xanthan gum, synthetic polymers, such as polyvinylpyrolidone, and the like. The preferred bulking agent is mannitol. The bulking agent is employed in an amount sufficient to provide a dry uniform, powdery substrate after lyophilization. Generally, the bulking agent is added to the aqueous emulsion at a concentration of from about 1mg/ml to about 10mg/ml preferably from about 3mg/ml to about 7mg/ml.

When the emulsion has been prepared, it is frozen and lyophilized in a conventional manner. Small aliquiots of the emulsion may be added to vials and lyophilized in situ, thus providing a product having a known amount of substrate and which may be reconstituted to an accurate concentration upon addition of a precise volume of water. Alternatively, the emulsion may be lyophilized in bulk and subsequently filled into vials or other suitable containers. It has been found that a powder filling aid, such as polyethylene glycol may facilitate such filling operations.

Utilizing the procedure described above, the lyophilzed substrate may contain from about 0.5 wt. % to about 5 wt. %, perferably from about 1.0 wt. % to about 2.5 wt. % of the triglyceride; from about 10 wt. % to about 40 wt. %, preferably from about 15 wt. % to about 30 wt. % of the surface active agent; from about 20 wt. % to about 70 wt. %, preferably from about 30 wt. % to about 50 wt. % of the bulking agent; and from about 20 wt. % to about 60 wt. % of the buffer.

The lyophilized substrates of this invention have been found to be stable and very reproducible. Initial turbidities of reconstituted products have been found to be controllable within ±10%. The product is very convenient in that the analyst simply mixes a predetermined amount of water with the lyophilized substrate. The substrate quickly disperses to form an emulsion which requires no further treatment prior to the addition of sample.

This invention is further illustrated by the following examples which are not intended to be limiting:

## EXAMPLE I

Tris-(hydroxymethyl)-aminomethane (60.56g) was dissolved in 950 ml. of deionized water. The temperature of the solution was adjusted to $25^{o}C$ and the pH adjusted to 9.0 with acid, at which time sodium desozycholate (24.88g) was added. The solution was diluted to 1000 ml. Two parts by weight of the buffer-surface active agent mixture was mixed with one part by weight of olive oil, and the mixture was emulsified by ultrasonication. The resulting concentrated emulsion was added to the buffer-surface active agent solution in the ratio 9.25 ml. of emulsion per liter of buffer-surface active agent mixture. This mixture was then further emulsified using high pressure homogenization. This pressure homogenized emulsion was then bulked with mannitol at a concentration of 50 milligrams per liter suitably diluted and lyophilized. The emulsion was frozen to $-40^{o}C$ and lyophilized at $10^{o}C$ per hour to a terminal temperature of $25^{o}C$ and maintained at this temperature for 12 hours.

## EXAMPLE II

The procedure of Example I is repeated in all essential details except triolein is substituted for olive oil and the emulsification is accomplished by a high speed blending. A stable lyophilized substrate is obtained.

## EXAMPLE III

The procedure of Example I is repeated in all essential details except sodium tauracholate is substituted of sodium desoxycholate and sucrose is substituted for mannitol. A stable lyophilized substrate is obtained.

- 7 -

## EXAMPLE IV

The procedure of Example I was repeated in all essential details except the emulsion was lyophilized in bulk and 0.816 grams of polyethylene glycol -4000 is added per gram of the dried product prior to filling into vials.

- 7 -

- 8 -

## CLAIMS

1. A substrate for use in a turbidimetric assay for lipase which comprises a lyophilized emulsion of from about 0.5 wt. % to about 5 wt. % of a lipase-hydrolyzable triglyceride; a pH-controlling amount of pH-7 to pH-10 buffer; from about 10 wt. % to about 40 wt. % of a surface active agent; and from about 20 wt. % to about 60 wt. % of a bulking agent.

2. The substrate of Claim 1 wherein the lipase-hydrolyzable triglyceride is a fatty acid triester of glycerol in which the fatty acid substituents each contain from about 16 to about 20 carbon atoms, and are employed at a concentration of from about 1.0 wt. % to about 2.5 wt. %.

3. The substrate of Claim 2 wherein the triglyceride is selected from the group consisting of olive oil, coconut oil, peanut oil, and sunflower seed oil.

4. The substrate of Claim 2 wherein the surface active agent is a bile salt.

5. The substrate of Claim 4 wherein the surface active agent is selected from the group consisting of physiological salts of desoxycholic acid, tauracholic acid, tauradesoxycholic acid, and chenodesoxycholic acid, and is employed at a concentration of from about 15 wt. % to about 30 wt. %.

6. The substrate of Claim 3 wherein the triglyceride is olive oil, the surface active agent is sodium desoxycholate, the buffer is tris-(hydroxymethyl)-aminomethane, and the bulking agent is mannitol.

7. A method for making a substrate for use in a turbidimetric assay for lipase, which comprises:

preparing an aqueous mixture containing a lipasehydrolyzable triglyceride at a concentration of from about 100 micromolar to about 425 micromolar; a surface active agent at a concentration of from about 0.001 molar to about 0.010 molar; and a pH-controlling amount of a pH-7 to pH-10 buffer;

emulsifying said aqueous mixture to form an emulsion which, when diluted to the concentration to which the final lyophilized product will be reconstituted, has an absorbance of from 0.5 to about 3.0 at 405nm in a 1 cm cell versus a water blank;

adding a bulking agent to said emulsion in an amount sufficient to yield a concentration of from about 1 mg/ml to about 10 mg/ml; and

freezing and lyophilizing said emulsion.

8.     The method of Claim 7 wherein the lipase-hydrolyzable triglyceride is a fatty acid triester of glycerol in which the fatty acid substituents each contain from about 16 to about 20 carbon atoms, and are employed at a concentration in the mixture of from about 200 micromolar to about 300 micromolar.

9.     The method of Claim 8 wherein the triglyceride is selected from the group consisting of olive oil, coconut oil, peanut oil, and sunflower seed oil.

10.    The method of Claim 8 wherein the surface active agent is a bile salt.

11.    The method of Claim 10 wherein the surface active agent is selected from the group consisting of physiological salts of desoxycholic acid, tauracholic acid, tauradesoxycholic acid, and chenodesoxycholic acid, and is employed at a concentration in the mixture of from about 0.004 molar to about 0.008 molar.

12.    The method of Claim 9 wherein the triglyceride is olive oil and the sodium desoxycholate, the buffer is tris-(hydroxymethyl)-aminomethane, and the bulking agent is mannitol.

0021572

European Patent
Office

**EUROPEAN SEARCH REPORT**

Application number

EP 80 30 1476

## DOCUMENTS CONSIDERED TO BE RELEVANT

| Category | Citation of document with indication, where appropriate, of relevant passages | Relevant to claim |
|---|---|---|
| | FR - A - 2 073 537 (BOEHRINGER MANNHEIM G.m.b.H.) <br><br> * Pages 5,6; claims 1-12 * | 1-7, 9-12 |
| | FR - A - 2 350 604 (E.I. DU PONT DE NEMOURS) <br> * Page 5, lines 3-18; pages 7,8, claims 1-17 * | 1-12 |
| A | US - A - 3 917 515 (J.M. GOLDBERG) <br><br> * Columns 5,6; claims 6-19 * | 1-6 |
| E,X | EP - A - 0 014 252 (BOEHRINGER MANNHEIM G.m.b.H.) <br><br> * Claims 1-15 * | 1-12 |

CLASSIFICATION OF THE
APPLICATION (Int. Cl ⁴)

C 12 Q 1/44

TECHNICAL FIELDS
SEARCHED (Int.Cl. ³)

C 12 Q   1/44
G 01 N 33/92

CATEGORY OF
CITED DOCUMENTS

X: particularly relevant
A: technological background
O: non-written disclosure
P: intermediate document
T: theory or principle underlying
   the invention
E: conflicting application
D: document cited in the
   application
L: citation for other reasons

&: member of the same patent
family,
corresponding document

The present search report has been drawn up for all claims

| Place of search | Date of completion of the search | Examiner |
|---|---|---|
| The Hague | 26.08.1980 | WALLINDER |

EPO Form 1503.1   06.78